# EUROPÄISCHE PATENTSCHRIFT

(11) **EP 1 408 857 B1**
(45) Veröffentlichungstag und Bekanntmachung des Hinweises auf die Patenterteilung: **23.11.2005**
(21) Anmeldenummer: 00940399.9
(22) Anmeldetag: 21.06.2000
(51) Int. Cl.: A61B 17/58, A61N 1/05

(54) **KNOCHENSCHRAUBE MIT VORRICHTUNG ZUR ELEKTROSTIMULATION**
BONE SCREW COMPRISING A DEVICE FOR ELECTROSTIMULATION
VIS A OS POURVUE D'UN DISPOSITIF D'ELECTROSTIMULATION

(30) Priorität: 23.06.1999 DE 19928449
(43) Veröffentlichungstag der Anmeldung: 21.04.2004
(73) Patentinhaber: GEOT Gesellschaft für Elektro-Osteo-Therapie GmbH, 80333 München (DE)
(72) Erfinder: LIEBRECHT, Felix, D-81371 München (DE); KRAUS, Werner, D-80333 München (DE); STEPHAN, Heribert, D-80689 München (DE)
(86) Internationale Anmeldenummer: PCT/EP2000/005780
(87) Internationale Veröffentlichungsnummer: WO 2001/000097

(56) Entgegenhaltungen:
- EP-A- 0 781 532
- DE-A- 2 215 111
- DE-A- 2 636 818
- DE-A- 4 230 181
- US-A- 5 292 252
- US-A- 5 725 377

## Beschreibung

Die vorliegende Erfindung betrifft eine Knochenschraube, die eine Vorrichtung zur Elektrostimulation enthält.

Es ist bekannt, dass das Gewebewachstum durch niederfrequenten, im wesentlichen sinusförmigen Wechselstrom angeregt werden kann [Verfahren nach Kraus und Lechner]. Um den Wechselstrom in dem zu stimulierenden Gewebebereich zur Einwirkung zu bringen ist es bekannt, eine elektrische Spule ("Aufnehmerspule") zu implantieren, mit deren Anschlüssen Elektroden ("Gewebeelektroden") gekoppelt sind, die an dem zu stimulierenden Gewebebereich appliziert werden. In der Aufnehmerspule wird durch eine externe, mit einem Schwingungsgenerator gekoppelte Primärspule eine elektrische Spannung induziert, die über die Elektroden auf den zu behandelnden Gewebebereich zur Einwirkung gebracht wird (DE-A-31 32 488).

Die Aumehmerspule kann z. B. in einem Marknagel untergebracht sein, dessen Oberfläche die Gewebeelektroden trägt (DE-A-26 36 818). Es ist auch bekannt, die Anschlüsse der Aumehmerspule über druckknopfartige Anschlussvorrichtungen mit Knochenschrauben zu koppeln, die zur Osteosynthese oder nur als Elektroden dienen (DE-A-39 42 735). Es ist auch eine Knochenschraube bekannt, die einen stabförmigen Permanentmagneten enthält.

Der vorliegenden Erfindung liegt die Aufgabe zugrunde, eine Knochenschraube mit einer Vorrichtung zur Elektrostimulation anzugeben, die einfach zu applizieren, sicher im Gebrauch und vielseitig anwendbar ist.

Aus dem Dokument EP-A-0 781 532 ist eine Hüftgelenkkopfprothese bekannt, deren Schaft einen inneren Hohlraum enthält und Löcher hat, die vom Hohlraum nach aussen führen. Am oberen Ende des Schaftes befindet sich ein Gewindeloch, das zum Füllen des Hohlraumes sowie zum Anschrauben eines Extraktionswerkzeuges dient und durch eine Einschlagschraube verschließbar ist. Der Hohlraum dient zur Aufnahme von Spongiosa, in die das den Schaft umgebende Gewebe durch die Löcher hinein wachsen soll. Um das Einwachsen des Gewebes zu fördern, wird die Einschlagschraube durch eine Elektrifiziervorrichtung ersetzt. Diese hat einen Sechskantkopf, ein sich an diesen anschliessendes Gewinde, das in das Gewindeloch passt, und einen in den Hohlraum reichenden Schaft. Der Schaft enthält eine Aufnehmerspule, die mit Elektroden verbunden ist, so dass im Hohlraum ein niederfrequenter elektrischer Wechselstrom induziert werden kann, der das Gewebewachstum stimuliert. Die Elektrifiziervorrichtung ist als Knochenschraube weder bestimmt noch geeignet, da sie den mechanischen Belastungen, denen eine Knochenschraube ausgesetzt ist, nicht gewachsen wäre. Außerdem ist das Maschinenschrauben-Gewinde, das in das Gewindeloch passen muss, sowohl hinsichtlich seiner Konfiguration als auch seiner Lage für eine Knochenschraube ungeeignet.

Aus den Dokumenten US-A-5 292 252 und US-A-5,725,377 sind Dentalimplantate bekannt, die einen Schaft mit Aussengewinde und am oberen Ende ein Gewindeloch zum Befestigen einer Zahnprothese hat. In das Gewindeloch ist eine StimulatorKappe eingeschraubt, die eine Batterie enthält. Bei einer Ausführungsform ist die Batterie mit einer im Schaft untergebrachten Spule verbunden, um in der Umgebung des Implantats ein elektrisches Gleichfeld zu erzeugen. Bei anderen Ausfühnmgsformen ist die Batterie einerseits mit dem Schaft und andererseits mit einer Elektrode verbunden, die sich an der Stirnseite der Stimulatorkappe befindet. Als Knochenschrauben, wie sie z. B. zur Befestigung von Osteosyntheseplatten und zum Zusammenschrauben von Knochenbruchstücken gebraucht werden, sind diese Dentalimplantate nicht geeignet.

Aus dem Dokument DE-A-42 30 181 ist ein Implantat zur Elektrostimulation von Gewebe bekannt, das eine Aufnehmerspule enthält, in der wie bei der Vorrichtung gemäß dem oben erwähnten Dokument DE-A-31 32 488 eine niederfrequente Wechselspannung induzierbar ist. Die Aufnehmerspule ist über flexible Leitungen mit Anschlüssen zum Kontaktieren von konventionellen Knochenschrauben oder mit Gewebeelktroden verbunden.

Der vorliegenden Erfindung liegt die Aufgabe zugrunde, eine Vorrichtung zur Elektrostimulation von Gewebe durch einen niederfrequenten elektrischen Wechselstrom anzugeben, die genau wie eine konventionelle Knochenschraube verwendet werden kann und zusätzlich zu deren mechanischer Funktion noch eine effektive Elektrostimulation des umgebenden Gewebes bewirkt.

Diese Aufgabe wird gemäß der vorliegenden Erfindung gelöst durch eine Knochenschraube mit
- einem rohrförmigen Schaft aus gewebeverträglichem, elektrisch leitfähigem Material, der an einem ersten Ende einen Kopf mit einer zum Ansetzen eines Drehwerkzeuges ausgebildeten Konfiguration aufweist;
- einem Endstück aus gewebeverträglichem, elektrisch leitfähigem Material, das an einem zweiten Ende des Schaftes angeordnet ist;
- einer elektrischen Isolierung zwischen dem Schaft und dem Endstück,
   einer im Schaft untergebrachten Aufnehmerspule, in welcher mittels einer externen Spule eine niederfrequente elektrische Wechselspannung induzierbar ist und welche mit einem ersten und einem zweiten Anschluss mit dem Schaft bzw. dem Endstück elektrisch gekoppelt ist, und
- mit einem Knochenschrauben-Außengewinde, das bei dem dem Kopf abgewandten Ende des Schaftes angeordnet ist.

Vorteilhafte Ausführungsformen der Erfindung weisen außerdem eines oder mehrere der folgenden Merkmale auf Das Endstück hat ein Vorderteil mit einem stabförmigen Ansatz, welcher in das zweite Ende des Schaftes isoliert eingesetzt ist. Zwischen dem Ansatz und dem Schaft befindet sich eine elektrisch isolierende, ringartige Anordnung und zwischen dem zweiten Ende des Schaftes und dem Vorderteil befindet sich ein elektrisch isolierendes Material. Das Endstück verjüngt sich mit zunehmendem Abstand vom Schaft. Das Außengewinde ist beim zweiten Ende des Schaftes mit Abstand von diesem oder im Anschluß an das zweite Ende des Schaftes an diesem oder am Vorderteil angeordnet. Die Knochenschraube einschließlich des Kerns der Aufnehmerspule und das Endstück sowie das Isoliermaterial weisen einen axialen Kanal auf, durch den ein dünner Führungsspieß gesteckt werden kann. Die Konchenschraube ist zweiteilig, d. h. sie enthält ein rohrförmiges Schraubenteil und einen in dieses einsteckbarten Einsatz, der das Endstück und die Aufnehmerspule enthält. Weitere Merkmale und Vorteile der Erfindung gehen aus der folgenden Beschreibung hervor.

Eine Knochenschraube gemäß einer ersten bevorzugten Ausführungform der Erfindung hat einen hohlen, rohrförmigen Schaft mit einem Kopf am einen Ende und einem selbstschneidenden Außengewinde am anderen Ende. Der Kopf hat, wie üblich, einen Innensechskant zum Einsetzen eines Drehwerkzeugs. In das andere Ende ist elektrisch isoliert ein Endstück eingesetzt, das sich zum Ende hin etwas, z. B. konisch oder spitzbogenförmig verjüngt. Im hohlen Schaft befindet sich eine Aufnehmerspule, deren Anschlüsse mit dem Schaft bzw. dem Endstück elektrisch gekoppelt sind. Der Zwischenraum zwischen dem Endstück und dem Ende des Schafts sowie das Innere des Schafts sind mit einer gewebeverträglichen Isoliermasse, wie einem Epoxyharz, ausgegossen.

Die vorliegende Knochenschaube lässt sich wie eine normale Knochenschraube leicht applizieren. Die Verbindung zwischen der Aumehmerspule und den als Elektroden dienenden Teilen, d. h. dem Schart und dem Endstück, ist mechanisch und elektrisch sicher, da sie sich in dem mit Isoliermasse vergossenen Inneren des Schraubenschafts befindet. Die Verjüngung des Endstücks erleichtert eine eventuelle spätere Extraktion.

Die Knochenschraube gemäß der Erfindung läßt sich für viele Indikationen anwenden, z. B. bei Hüftkopfnekrosen, Osteochondrosis dissecans, Wirbelversteifung und in Kombination mit weiteren, normalen Knochenschrauben bei der Fixierung einer medialen Schenkelhalsfraktur.

Im folgenden wird die Erfindung anhand der Zeichnung näher erläutert. Es zeigen:
- Figur 1: einen Längsschnitt einer Knochenschraube gemäß einem ersten Ausführungsbeispiel der Erfindung;
- Fig. 2: einen Längsschnitt einer Knochenschraube gemäß einem zweiten, derzeit bevorzugten Ausführungsbeispiel der Erfindung;
- Fig. 3: einen Längsschnitt einer weiteren bevorzugten Ausführungsform der Erfindung, und
- Fig. 4: einen vereinfachten Längsschnitt einer zweiteiligen Ausführungform der Erfindung.

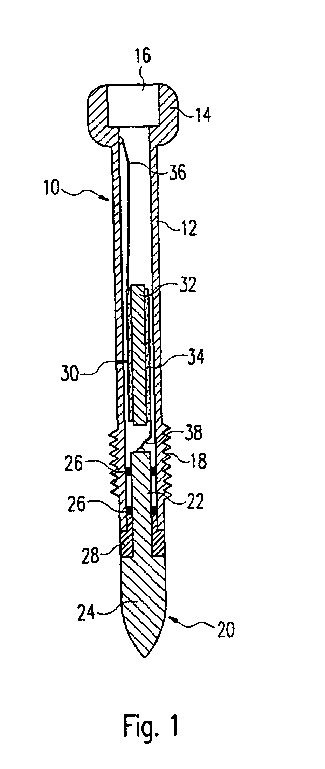

Die in Fig. 1 dargestellte Knochenschaube 10 hat einen rohrförmigen, beidseits offenen Schaft 12 aus gewebeverträglichem Metall. Am einen Ende des Schaftes befindet sich ein integraler Kopf 14. Der Kopf hat eine sechseckige Ausnehmung 16 oder irgend eine andere Konfiguration zum Ein- oder Ansetzen eines Drehwerkzeuges. An dem dem Kopf 14 abgewandten Ende des Schafts 12 oder in dessen Nähe hat der Schaft ein integrales, selbstschneidendes Außengewinde 18.

In das dem Kopf 14 abgewandte Ende des Schafts 12 ist ein Endstück 20 aus gewebeverträglichem Metall elektrisch isoliert eingesetzt. Das Endstück 20 hat einen stabförmigen Ansatz 22 und ein sich verjüngendes Vorderteil 24, dessen dem Schaft 12 zugewandtes Ende den gleichen Außendurchmesser wie der Schaft 12 hat. Der Ansatz 22 steckt im hohlen Schaft 12 und ist gegen diesen durch zwei dünne Ringe 26 aus Kunststoff elektrisch isoliert. Zwischen dem Vorderteil 24 und dem Schaft 12 verbleibt ein Zwischenraum, der mit gewebeverträglichem Kunststoff 28, wie Epoxyharz, ausgegossen ist. Die Oberfläche der Kunststottisolierung 28 fluchtet mit der Oberfläche des Schaftes 12 und dem anschließenden Ende des Endstücks [24].

Im Inneren des Schafts 12 ist eine Aufuelunerspule 30 untergebracht, die einen Magnetkern 32 aus magnetisch weichem Material und eine Drahtwicklung 34 enthält. Die Wicklung 34 hat zwei Anschlüsse 36, 38, die mit dem Schaft 12 bzw. dem Endstück 20 elektrisch gekoppelt, z. B. durch Punktschweissen verbunden sind. Der Schaft 12 und das Endstück 20 dienen also als Gewebeelektroden. Der verbleibende Hohlraum im Schaft 12 mit Ausnahme der Ausnehmung 16 ist mit Kunstharz ausgegossen, das der Übersichtlichkeit halber nicht dargestellt ist. Der Hohlraum ist über die Öffnung des Schafts 12 im Kopf 14 zugänglich.

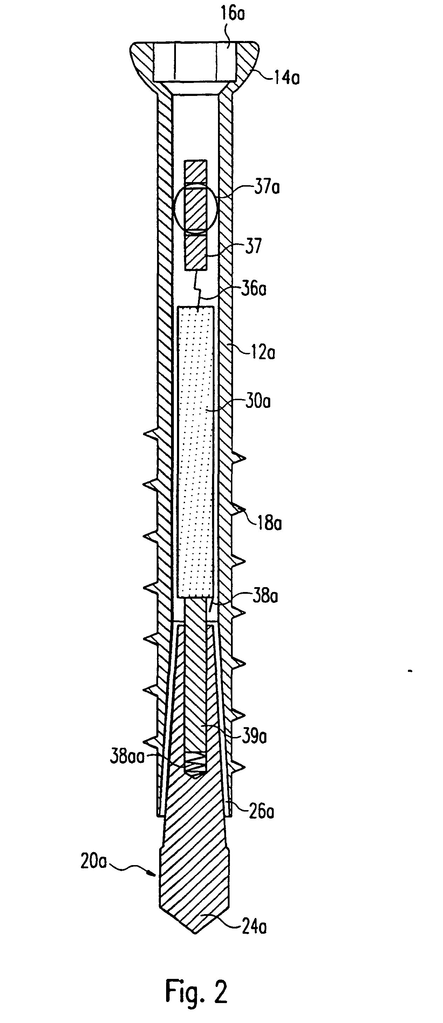

Die Knochenschraube gemäß Fig. 2 entspricht im wesentlichen der gemäß Fig. 1, entsprechende Teile sind mit denselben Bezugszahlen bezeichnet, denen ein"a" angefügt ist. Es genügt daher, die Unterschiede zu beschreiben: Der Anschlußdraht 36a der Aufnehmerspule 30a ist an einem Metallzylinder 37 angeschweißt, das zwei Querbohrungen hat, in denen eine ringförmige Kontaktfeder 37a gehaltert ist, die an der Innenwand des Schaftes 12a anliegt. Der andere Anschluß 38a der Aufnehmerspule 30a ist an einem Teil 39a des Magnetkerns, der aus der Spulenwicklung herausragt, angeschweißt. In einer Bohrung an der Stirnseite des Magnetkerns steckt ein Stück Federdraht 38aa, das auf Grund seiner Federkraft einen sicheren elektrischen Kontakt mit der Innenwand des Schaftes 12a macht. Die Isolierung zwischen dem Schaft 12a und dem Endstück 20a besteht aus einer Epoxyharzschicht 26a.

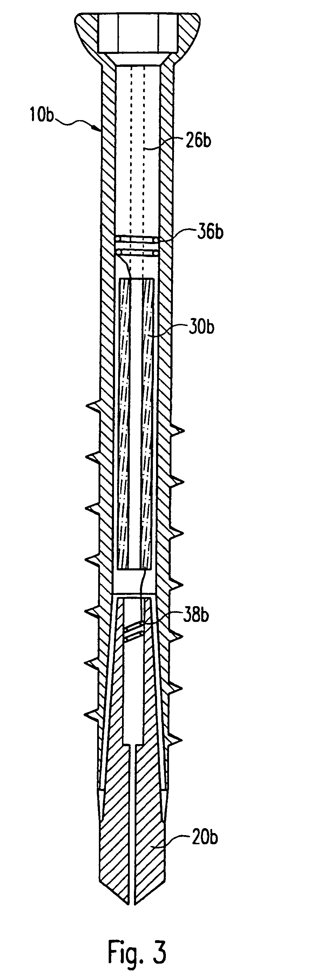

Die Ausführungsform gemäß Fig. 3 entspricht weitgehend der gemäß Fig. 2 mit der Ausnahme, daß die Knochenschraube 10b einen durchgehenden, axial verlaufenden Kanal aufweist, durch den ein dünner Führungsspieß gesteckt werden kann. Der Kanal geht also durch die Isolierstoffüllung, wie bei 26b angedeutet ist, durch die auf einen rohrförmigen Magnetkern gewickelte Aufnehmerspule 30b und das Endstück 20b. Die Anschlußdrähte 36b, 38b haben wendelförmige federnde Enden, die den axialen Kanal frei lassen.

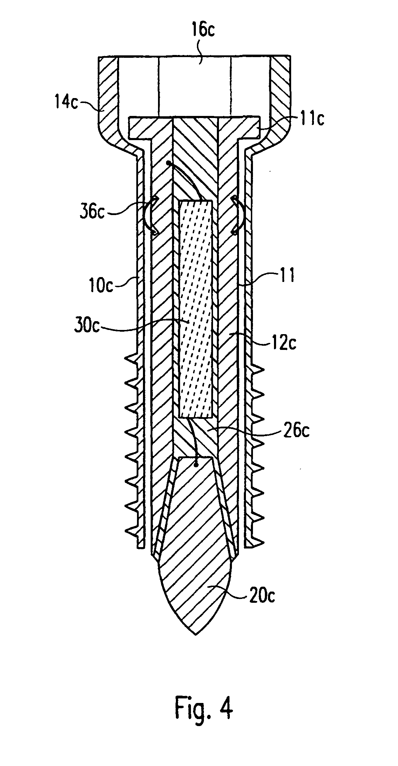

Die Ausführungsform gemäß Fig. 4 ist ähnlich der gemäß Fig. 1, sie enthält jedoch zwei Teile, nämlich einen rohrförmigen Knochenschraubenteil 10c und einen Einsatz 11. Der Einsatz 11 hat einen flachen Kopf 11c, der in die Ausnehmung 16c paßt, und einen Schaft 12c, der in den hohlen Schaft des Knochenschraubenteils 10c paßt. Im Schaft 12c befindet sich eine Aufnehmerspule 30c. An dem dem Kopf 11c abgewandten Ende des Schaftes 12c ist ein Endstück isoliert gelagert. Die Anschlüsse der Aufnehmerspule 30c sind mit dem Schaft 12c bzw. dem Endstück 20c verbunden. Im Schaft 12c sind Kontaktfedern gelagert, die eine elektrische Verbindung zwischen dem Schaft 12c und dem Teil 10c sicherstellen. Bei Benutzung wird zuerst der Knochenschraubenteil 10c mit einem Führungsspieß implantiert und dann wird der "Elektro"-Einsatz 11 in den hohlen Knochenschaubenteil eingesteckt.

Die Metallteile können aus Titanmetall oder irgend einem anderen bekannten gewebeverträglichem Metall bestehen. Einer metallischen Ausführung ist ein mit einem elektrisch leitfähigen Material versehenes Isoliermaterial äquivalent. Z. B. kann das Endstück 20 aus mit Titan o. dgl. bedampfter Keramik bestehen.

Bei abgewandelten Ausführungsformen reicht das Gewinde bis zum Ende des Schaftes 12 oder es befindet sich am Endstück 20.

## Patentansprüche

1. Knochenschraube mit
- einem rohrförmigen Schaft [12] aus gewebeverträglichem, elektrisch leitfähigen Material,
- einer ersten und einer zweiten Gewebeelektrode, von denen die erste aus dem Schaft [12] besteht,
- einer elektrischen Isolierung [28] zwischen der zweiten Gewebeelektrode.[20] und dem Schaft [12],
- einer im Schaft [12] untergebrachten Aufnehmerspule [30], in welcher mittels einer externen Spule eine niederfrequente Wechselspannung induzierbar ist und welche mittels eines ersten und eines zweiten Anschlusses [36, 38] mit dem Schaft [12] bzw. der zweiten Gewebeelektrode [30] elektrisch gekoppelt ist,
**dadurch gekennzeichnet, daß**
- der Schaft an einem ersten Ende einen Kopf [14] mit einer zum Angreifen eines Drehwerkzeuges geeigneten Konfiguration aufweist,
- die zweite Gewebeelektrode [20] an einem zweiten, dem Kopf abgewandten Ende des Schaftes [12] angeordnet ist,
- ein selbstschneidendes Außengewinde [18] vorgesehen ist.

2. Knochenschraube nach Anspruch 1, **dadurch gekennzeichnet, dass** sich die aus dem rohrförmigen Schaft herausragende zweite Gewebeelektrode [20] mit zunehmendem Abstand vom Schaft [12] verjüngt.

3. Knochenschraube nach Anspruch 1, **dadurch gekennzeichnet, dass** das Außengewinde an der aus dem rohrförmigen Schaft herausragenden zweiten Gewebeelektrode angeordnet ist.

4. Knochenschraube nach Anspruch 1, **dadurch gekennzeichnet, dass** sie einen durchgehenden, axial verlaufenden Kanal hat.

5. Knochenschraube nach Anspruch 1, **gekennzeichnet durch** einen rohrförmigen, an beiden Enden offenen Knochenschraubenteil [10c], der einen Kopf zum Ansetzen eines Drehwerkzeuges aufweist, und **durch** einen in diesen einsteckbaren Einsatz [11], der die Aufnehmerspule [30c] und die zweite Gewebeelektrode [20c] enthält, die, wenn der Einsatz [11] in den Knochenschraubenteil [10c] eingesetzt ist, aus dem dem Kopf abgewandten Ende des Knochenschraubenteils herausragt.

6. Knochenschraube nach Anspruch 1, **gekennzeichnet durch** eine Kontaktfeder [37a, 38a, 36b, 38b, 36c], an mindestens einem der Anschlüsse der Aufnehmerspule.

## Claims

1. A bone screw comprising
- a tubular shaft (12) made of a tissue-compatible, electrically conductive material,
- a first and a second tissue electrode, the first thereof being constituted by the shaft (12),
- an electric insulation (28) between the second tissue electrode (20) and the shaft (12),
- a pick-up coil (30) housed within the shaft (12), and to which a low-frequency alternating voltage may be induced by means of an external coil, and which is electrically coupled to the shaft (12) or the second tissue electrode (30) by means of a first and a second connection (36, 38),
**characterized in that**
- the shaft has a head (14) on a first end, said head (14) having a configuration suited for engagement of a turning tool,
- the second tissue electrode (20) is arranged on a second end of the shaft (12) facing away from the head,
- a self-tapping external thread (18) is provided.

2. The bone screw according to claim 1, **characterized in that** the second tissue electrode (20) protruding from the tubular shaft tapers with increasing distance from the shaft (12).

3. The bone screw according to claim 1, **characterized in that** the external thread is arranged on the tissue electrode protruding from the tubular shaft.

4. The bone screw according to claim 1, **characterized in that** it has a continuous, axially extending channel.

5. The bone screw according to claim 1, **characterized by** a tubular bone screw member (10c) open at both ends, which has a head for applying a turning tool, and by an insert (11) insertable therein, which contains the pick-up coil (30c) and the second tissue electrode (20c) which, when the insert (11) is inserted into the bone screw member (10c), protrudes from the end of the bone screw member facing away from the head.

6. The bone screw according to claim 1, **characterized by** a contact spring (37a, 38a, 36b, 38b, 36c) on at least one of the pick-up coil connections.

## Revendications

1. Vis d'os comprenant
- un arbre (12) en forme de tube en matériau électriquement conducteur, supporté par les tissus,
- une première et une deuxième électrode de tissu dont la première se constitue de l'arbre (12),
- une isolation électrique(28) entre la deuxième électrode de tissu (20) et l'arbre (12),
- une bobine capteur (30) logée dans l'arbre (12), dans laquelle peut être induite une tension alternative basse fréquence au moyen d'une bobine extérieure et qui est électriquement couplée par un premier et un deuxième raccordement (36, 38) à l'arbre (12) ou à la deuxième électrode de tissu (30),
**caractérisée en ce que**
- l'arbre présente une tête (14) avec une configuration adaptée à l'engagement d'un outil rotatif,
- la deuxième électrode de tissu (20) est disposée à une deuxième extrémité de l'arbre (12) détournée de la tête,
- un filetage extérieur auto-taraudant (18) est prévu.

2. Vis d'os selon la revendication 1, **caractérisée en ce que** la deuxième électrode de tissu (20) dépassant de l'arbre en forme de tube se rétrécit en s'éloignant de l'arbre (12).

3. Vis d'os selon la revendication 1, **caractérisée en ce que** le filetage extérieur est disposé à la deuxième électrode de tissu dépassant de l'arbre en forme de tube.

4. Vis d'os selon la revendication 1, **caractérisée en ce qu'**elle possède un canal axial, continu.

5. Vis d'os selon la revendication 1, **caractérisée par** une partie de vis d'os (10c) en forme de tube, ouverte aux deux extrémités, qui présente une partie pour monter un outil rotatif, et par un insert (11) pouvant s'enficher dans celle-ci, qui contient la bobine capteur (30c) et la deuxième électrode de tissu (20c), qui, lorsque l'insert (11) est disposé dans la partie de vis d'os (10c), dépasse de l'extrémité de la partie de vis d'os détournée de la tête.

6. Vis d'os selon la revendication 1, **caractérisée par** un ressort de contact (37a, 38a, 36b, 38b, 36c) en au moins l'un des raccords de la bobine capteur.
